# EUROPEAN PATENT SPECIFICATION

(11) **EP 4 456 856 B1**
(45) Date of publication and mention of the grant of the patent: **30.07.2025**
(21) Application number: 22835904.8
(22) Date of filing: 21.12.2022
(51) Int. Cl.: A61H 7/00

(54) **KIT FOR IMPROVING THE DISTURBANCES ASSOCIATED WITH THE INFLAMMATION OF ADIPOSE TISSUE, WITH THE PHLEBOLYMPHATIC PATHOLOGIES AND FOR MUSCULAR REHABILITATION IN GENERAL**
KIT ZUR VERBESSERUNG DER STÖRUNGEN IN ZUSAMMENHANG MIT ENTZÜNDUNGEN DES FETTGEWEBES MIT PHLEBOLYMPHATISCHEN ERKRANKUNGEN UND ZUR MUSKELREHABILITATION IM ALLGEMEINEN
KIT POUR AMÉLIORER LES PERTURBATIONS ASSOCIÉES À L'INFLAMMATION DU TISSU ADIPEUX, AVEC LES PATHOLOGIES PHLÉBOLYMPHATIQUES ET POUR LA RÉÉDUCATION MUSCULAIRE EN GÉNÉRAL

(30) Priority: 28.12.2021 IT 202100032756
(43) Date of publication of application: 06.11.2024
(73) Proprietor: Fenix Group S.r.l., 65015 Montesilvano (PE) (IT)
(72) Inventor: CAVALLETTI, Gianluca, 65015 Montesilvano (PE) (IT)
(74) Representative: Fiammenghi, Eva
(86) International application number: PCT/IB2022/062588
(87) International publication number: WO 2023/126778

(56) References cited:
- WO-A1-2020/148624
- US-A1- 2020 054 890
- US-A1- 2021 146 119

## Description

### Technical field

The present patent application for an invention generally relates to the field of aesthetic medicine, sports medicine and phlebo-lymphology.

The invention is applicable to any field where such a type of device can be advantageously used, but preferably it relates to the field of the treatment of the various forms of cellulite and lipolymphedema.

### Prior art

As is known, cellulite manifests itself from an aesthetic point of view in the form of irregularities on the surface of the epidermis and is scientifically referred to as edematous-fibrosclerotic panniculopathy.

Cellulite indicates an alteration of the subcutaneous tissue rich in fat cells and is characterized precisely by the increase in the volume of fat cells, where excess liquids accumulate in the intracellular spaces, which are left over from the body's biochemical processes. The balance of the venous and lymphatic systems is modified by a slowing down of blood flow and fluid retention by the tissues.

The onset of cellulite, even if basically everything can be traced back to an alteration of the microcirculation, can be considered dependent on various factors which often add up to each other. Some of these factors cannot be eliminated and are therefore defined as primary as they are due to sex, race or familiarity, others are defined as secondary as they are connected to certain phases of life, particular pathologies or the intake of drugs and others still aggravating factors are defined as well as poor diet or sedentary lifestyle and which could certainly be controlled by adopting a different lifestyle. Since the 1970s, cellulite is no longer classified as a generic imperfection, but as an autonomous pathological condition.

Treatment of cellulite includes surgical methods and non-invasive methods.

The operation to surgically eliminate cellulite is generically called liposuction and involves the effective removal of fat deposits in limited and fairly extensive body areas, also with the use of general anesthesia. Obviously it is a technique, which although valid, is very invasive and therefore indicated only in the most serious cases.

Leaving aside the treatments with anti-cellulite creams, which are not directly part of the present invention, there are many non-invasive treatments to cure cellulite and which consist of medical techniques that use equipment such as cavitation, ultrasound, radiofrequency, electrolipolysis, cryosculpture, laser therapy, ozone therapy, pressotherapy, lymphatic drainage and magnetotherapy. These techniques are widely practiced both in the professional field and directly by the patient and are more or less effective in resolving or at least reducing this imperfection.

Starting from the consideration that for years the technique of dermo-suction has been used, thanks to which the tissues were sucked up and placed in traction by means of cylinders, this technology has gradually evolved using less bloody techniques for the purpose which, both in the professional field and directly by the user, were based on the well-known compressive micro-vibration technology of the tissues, but which resulted to be variously effective in solving or at least reducing the type of imperfection reported above.

Therefore, the main object of the present invention consists in general in realizing a kit also based on the well-known technology of compressive micro-vibration of the tissues, which is able to act even more effectively on the improvement of this imperfection, compared to those existing in the prior art and that adopt this technology.

A further object of the present invention is to provide to all those who carry out a professional activity, relating to the treatment of this type of blemish, a medical kit which is capable of implementing in synergy at least two of the techniques previously mentioned, in order to be able to effectively extend the treatment to one type of disorder or better synergistically to several types of disorders.

Therefore, the object of the present invention consists in general in realizing a device designed to solve the above problems, as will result from the detailed description of an exemplifying and non-limiting embodiment thereof, illustrated below.

### Summary of the invention

The present patent application for industrial invention is intended to describe and claim a kit provided with at least a new and alternative solution to the solutions known to date and in particular it aims to obviate one or more of the drawbacks or problems referred to above and/or to meet one or more needs perceived in the art and in particular deducible from the above.

For this purpose, the inventors have developed the medical kit according to claim 1, capable of realizing the benefits deriving from a combined magnetotherapy treatment, am electrostimulation treatment and a compressive microvibration treatment of the tissues and all combined with an effective thermographic control. In fact, said inventive kit consists of a magnetotherapy device with compressive microvibration, at least an electrostimulator device and a digital thermographic camera.

Specifically, said kit can be advantageously used to improve di sorders associated with vascular hemodynamics, increase in interstitial fibrous tissue, lymphatic stasis and lymphedema, and painful conditions deriving from edematous conditions and post-traumatic and rehabilitative physiotherapy.

More specifically, said inventive kit comprises a base element provided with at least a recess for housing at least a handpiece, at least a screen, preferably a touch-screen, capable of providing a communication interface between the user and the computer terminal included in such base element. Said base element is connected via an electrical connection cable or via a battery system with Wi-Fi or Bluetooth radio connection, to said handpiece, inside which a rotor is housed consisting of multiple balls having a particular internal and external shape associated with calibrated elastomeric features, capable of transferring pressure to the body area being treated in a controlled manner by virtue of their particular viscoelastic nature. Specifically, a fundamental feature of the invention concerns the fact that the system is suitable for ensuring that by placing the handpiece on the dermis only with its own weight, the mechanical resistance encountered by the rotor balls while sliding on the patient's tissue is detected by means of a dedicated system of pressure and speed sensors, thus detecting the type of edematous or fibrous tissue with which the balls come into contact and thus independently regulating the intensity of the device according to the resistance by means of a dedicated software.

Specifically, a particularly fundamental feature, according to the present invention, relates to the fact that said calibrated elastomeric features of the balls, i.e. of each row containing balls having the same viscoelastic properties represents a means by which to obtain compressive harmonic waves determined on the body area being treated in addition to the classic pulsating compression effect already existing in the art. Even more specifically, said compressive microvibration technology of the tissues implemented by the formation of compressive harmonic waves on the body area being treated is due to a hypercompressive effect, which exploits the muscle as active resistance when said body tissue comes into contact cyclically with a row of balls having high rigidity during the rotation of the rotor. Therefore, it is intuitive to consider that the frequency, duration and amplitude of said compressive harmonic waves derive directly also from the arrangement of said rows of balls having high rigidity between the rows of balls having viscoelastic properties.

Another innovative feature of said inventive kit consists in that the benefits deriving from the aforementioned technology of compressive microvibration of the tissues are effectively implemented by combining it with the physical therapy of magnetotherapy and all this by designing said handpiece in order to accommodate a specially conceived solenoid capable of emitting a suitable electromagnetic field to implement a magnetotherapy treatment in synergy. In fact, magnetotherapy seems to be able to stimulate tissue regeneration, due to the restoration of a cellular biochemical balance exclusively and effectively for therapeutic and curative purposes. For example, it seems to be effective as an adjuvant treatment against cellulite, precisely because it acts directly on the adipocytes, allowing a strong metabolic stress and locally of the blood circulation to be induced, reducing water retention and inflammation that accompany cellulite itself.

A further fundamental feature of the present inventive kit consists in the presence of the electrostimulator included in said base element, suitable for carrying out a targeted electrostimulation treatment in the vicinity of the object body area and in conjunction with the magnetotherapy and microvibration treatment. The positioning of electrodes, whether they are self-adhesive electrodes, pre-gelled electrodes or an electrostimulator band, on areas of the body subjected to compressive microvibration treatment represents a substantial feature of the present invention, as it is inventively associated with the already referred feature of the production of compressive harmonic waves determined on the body area being treated. In particular, the previously mentioned hypercompressive effect, determined by the presence of at least a row of rigid balls between the rows of balls having certain viscoelastic properties, allows by virtue of said dedicated software which regulates the rotor speed parameters and the type of pulse, frequency, duration, intensity and latency, to make the instant in which said hypercompression operated by the row of rigid balls takes place on the tissue coincide with the instant in which the electric impulse is generated. In particular, said synchronism allows for an involuntary contraction of the muscle to be produced, suitable for amplifying the active resistance of the muscle itself, at the instant in which the hypercompressive effect occurs and therefore allows for the genesis of amplified compressive harmonic waves which propagate effectively also to the neighboring areas.

A further and no less fundamental inventive feature relevant to the inventive purposes of the present kit concerns the possibility of implementing a thermographic technique through the presence of a digital thermographic camera connected to the computer terminal housed in said base element. The mapping of the portions of the body before, during and at the end of the treatment session, obtained by passing said digital thermographic camera over the patient's body areas, allows to visually highlight, with different colors, the thermal differences present on the body areas. In this way it is possible to differentiate normothermic areas, with normal blood circulation, from more hypothermic areas, where blood circulation is impaired or where there is poor circulation and therefore the presence of cellulite in its various stages.

A further aspect, on the sidelines of all the technical solutions described up to now, but of particular relevance in the implementation of the invention, concerns the creation and development of a multiplicity of programs used in the data processing system and which create a software specially developed by the inventors, capable of being able to best and effectively perform all the functions deriving from the technical features described up to now.

Said dedicated software is able to process the input data coming from the sensors placed on the handpiece and to manage the output data for regulating the speed of the rotor, which is regulated both by synchronizing it with the electrical impulses emitted by the electrostimulator and by regulating it on the basis the presence of variously fibrotic areas through a fine feedback mechanism, which also provides for the intervention of the operator able to vary the automatic setting of the system. In other words, said device inventively presents a mechanism for adjusting the intensity of the stimuli emitted by the electrostimulator as well as of the associated speed of the rotor according to the resistance encountered on the tissue and therefore relating to the presence of more or less incompressible fibrous areas and which represent an unequivocal sign of cellulite areas.

It is therefore a specific object of the present invention to implement an inventive medical kit including the relative dedicated software, capable of realizing the benefits deriving from a combined magnetotherapy treatment, a compressive microvibration treatment of the and electrostimulation and all assisted by the thermographic technique in the treatment or alleviation of aesthetic disorders resulting from cellulite.

A further and more general object is to be able to implement a kit capable of being advantageously used in the improvement of disorders associated with vascular hemodynamics, the increase in interstitial fibrous tissue, lymphatic stasis and lymphedema, to painful states resulting from edematous conditions.

Other features of the present invention are described in the following detailed description of one or more specific embodiments thereof, protected by the various dependent claims.

### Brief description of the drawings

The foregoing advantages, as well as other advantages and features of the present invention, will be illustrated with reference to the accompanying drawings, which are to be considered purely illustrative and not limiting or binding to the effects of the present patent application, in which:
- FIGURE 1 is a perspective view of the components of the inventive kit;
- FIGURE 2A is a front view of a handpiece forming part of the kit;
- FIGURE 2B is a sectioned front view of the handpiece forming part of the kit;
- FIGURE 3A is an exploded view of the handpiece that is part of the kit;
- FIGURE 3B shows in detail the exploded view of the rotor installed in the handpiece according to the invention;
- FIGURE 4 shows in detail the exploded view of the inductor and the plug of the handpiece according to the invention;
- FIGURE 5 shows in detail the exploded view of the interface and of the isolating and clamping support means of the handpiece cable according to the invention;
- FIGURE 6A shows in detail the perspective view of an elastomeric ball;
- FIGURE 6b is the plan view of the elastomeric ball.

### Detailed description of the invention

It will be immediately apparent that innumerable variations and modifications (for example relating to shape, dimensions, arrangements and parts with equivalent functionality) may be made to what has been described without departing from the scope of the invention as appears in the appended claims.

In fact, with the present invention, a new and/or alternative solution to the solutions known thus far is proposed and in particular it is proposed to remedy one or more of the drawbacks or problems referred to above, and/or to satisfy one or more requirements felt in the art, and in particular inferrable from the foregoing.

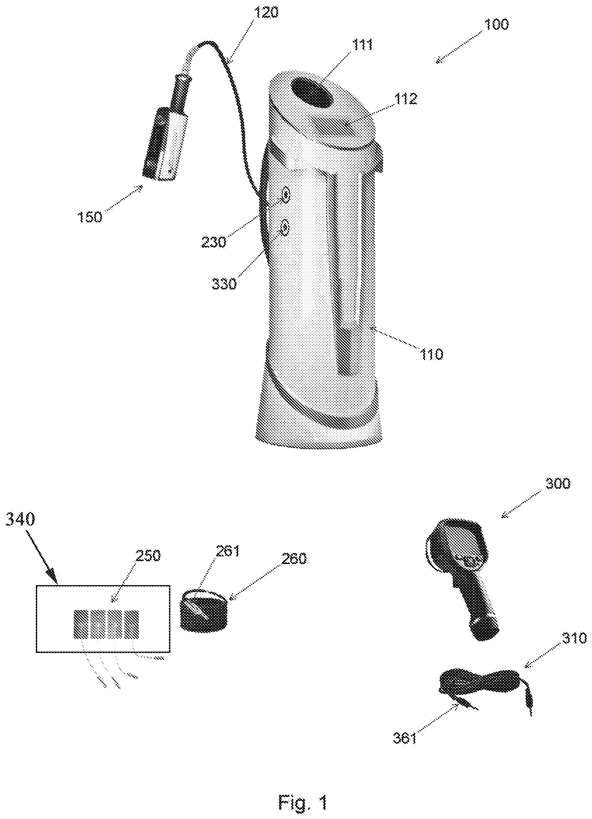

As shown in the detail of Fig.1, a kit is shown for the treatment of inflammation of the adipose tissue, phlebolymphatic pathologies and for muscle rehabilitation in general comprising a magnetotherapy device with compressive microvibration 100, an electrostimulator device and a digital thermographic camera 300.

More specifically, said magnetotherapy device with compressive microvibration 100 consists of a base element 110 provided with at least a recess 111 for housing at least a handpiece 150, at least a screen 112, preferably a touch-screen, capable of providing a communication interface between the user and the computer terminal included in such base element 110. Said base element 110 is connected via an electrical connection cable 120 or via any wireless system, to said handpiece 150, inside which a rotor 200 is housed consisting of multiple balls having an internal and external conformation suitable for achieving calibrated elastomeric features, able to transfer energy to the body area being treated in a controlled manner, by virtue of their particular viscoelastic nature.

In particular, the different geometric configuration of the balls is aimed at obtaining a particular and pre-established effect on the patient's skin.

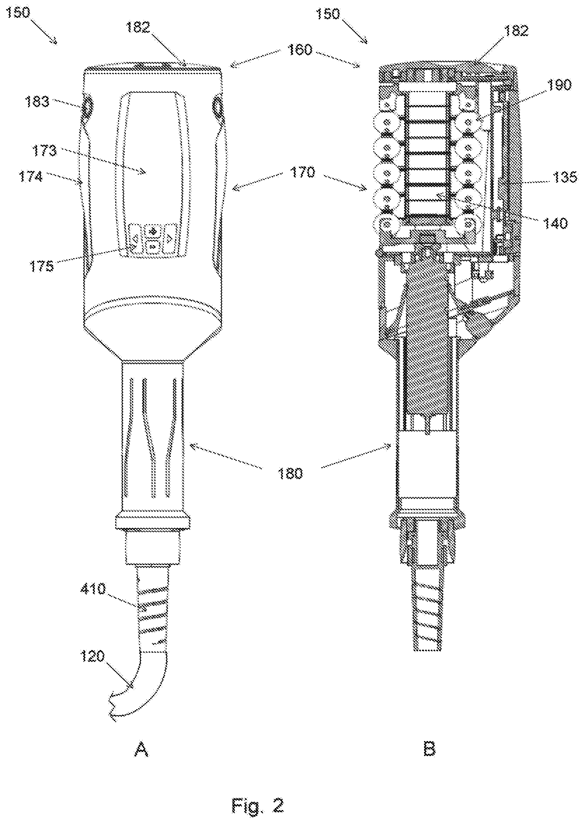

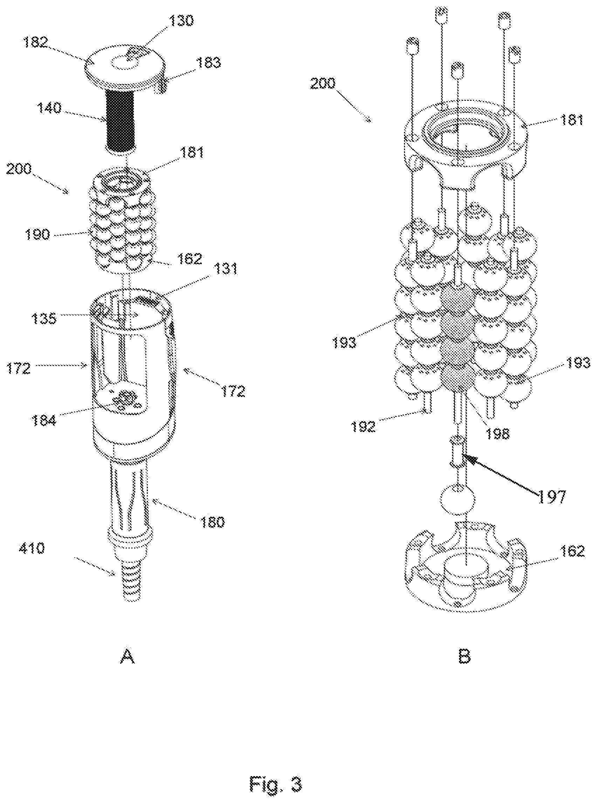

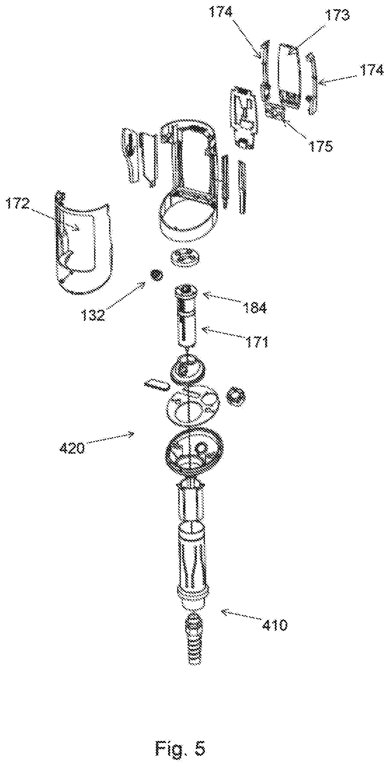

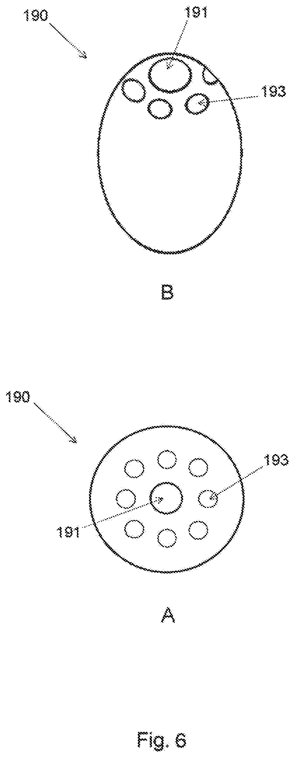

As shown in Figs. 2A-B, said handpiece 150 consists of a head 160, a body 170 and a handle 180. Said body 170 has a recess suitable for housing said rotor 200 as shown in Figs. 3A-B, is shaped like a hollow cylinder, which on the lateral surface has a plurality of balls 190 inserted through the longitudinal central channels 191 (see Fig. 6) on sliding bushes 197 (see Fig. 3B) so as to allow free rotation on themselves, in which each pin 192 is common to at least four balls 190 and in which the axis of rotation of the balls 190 and of the rotor 200 coincides with that of insertion into the recess of the handpiece 150 and in which said pins 192 are constrained respectively to a head ring nut 181 and to a ring nut of the handle 162. Said ring nut of the handle 162 comprises a central fitting system adapted to be inserted on a rotation mechanism 184 shown specifically in Fig. 5, forming part of an electric motor 171 housed inside said handle 180 and wherein said electric motor 171 is connected by means of isolating support means 420 and tightening means 410 to said electrical connection cable 120 connected in turn to said computer terminal compressed inside the base element 110.

The rotation of the rotor 200 is such as to cause the passive rotation of each individual ball 190 on itself, every time it slides on the patient's tissue, through a rectangular opening 172 shown in Figs. 3 and 5 and made on said body 170 of the handpiece 150. As shown in Figs. 2A and 5, on the body 170 in a position opposite to that of the rectangular opening 172, an interface is made comprising a display 173, a plurality of indicators 174 and a plurality of buttons 175.

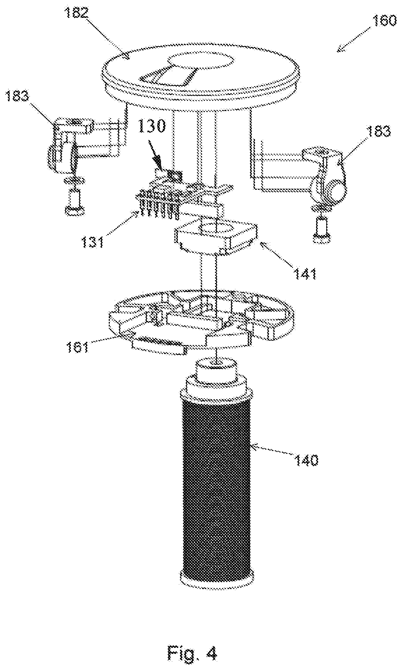

With reference to Fig. 4, the head 160 comprises a flange 161, in which one face of the same is adapted to allow the rotation of said head ring nut 181 while the other face is constrained to a cap 182 of the handpiece. Said cap 182 is connected to the head 160 of the handpiece 150 through at least a common pair of reversible connection systems 183 and therefore capable of being removed to allow easy extraction of the rotor 200 after each session to replace it or for related sanitization.

A fundamental feature of the present invention is that of comprising said balls 190 made of an elastomeric material having different degrees of elasticity and which, as shown in detail in Figs. 2, 3 and 6A-B, have a particular internal structure consisting of multiple longitudinal internal channels 193 made in parallel around said longitudinal central channel 191 or geometric shapes suitable for reproducing a particular elastic effect.

The association of said elastomeric property with the presence of said longitudinal internal channels 193 is adapted to give said balls 190 well-defined viscoelastic properties and exploited to absorb in a calibrated manner part of the pressure transferred by the handpiece 150 on the patient's body area being treated. As shown in Fig. 3, another innovative feature derives from the fact that the creation of rows with balls 190 each having the same viscoelastic property associated with the presence in the rotor of at least a row of rigid balls 198 (see Fig. 3B) without said longitudinal internal channels 193 and therefore capable of exerting a hypercompressive effect with respect to the analogous balls 190, gives the inventive device the ability to produce specific compressive harmonic waves amplified on the body area being treated, in addition to the classic compressive effect already existing in the art. Therefore, it is intuitive to consider that the frequency, duration and shape of said compressive harmonic waves also derive directly from the arrangement of said rows of balls 190 having viscoelastic properties associated with the presence of at least a row of rigid balls 198.

It forms a further specific feature of the present invention that of comprising in the cavity of said rotor 200 an inducer device 140, specifically shown in Figs. 2B, 3A and 4, consisting of at least a solenoid with a cylindrical shape, which engages a rotary bearing 141 via one end which in turn is constrained to said head ring nut 181. The presence of said inducer device 140 inside the handpiece 150 is an aspect which further characterizes the present invention, since it allows obtaining a combined magnetotherapy treatment and a modified compressive microvibration treatment of the tissues.

Another specific feature of the present invention shown in Figs. 2A and 3A is that of making a pressure sensor 130 on the cap 182 of the handpiece adapted to detect the pressure of the rotor 200 exerted by the balls 190 on the tissue of the patient undergoing therapy. Also, said pressure sensor 130 results, through a connection device 131 consisting of a connector provided with a plurality of pins (Figs. 2 and 4), in connection with an electronic control unit 135 shown in Fig. 2B.

A further specific feature of the present invention as shown in Fig. 5, is to provide said handpiece with a revolution sensor 132 located near the rotation mechanism 184 or via an encoder positioned on the rotary axis of the motor, and adapted to detect the revolutions of the rotor in real time and therefore to send the data to said electronic control unit 135, which it is controlled by the computer terminal by means of a wiring system, which passes through said electrical connection cable 120.

Specifically, a fundamental feature of the invention relates to the fact that the entire system inventively presents a mechanism for adjusting the intensity of the device according to the resistance encountered on the tissue and therefore relating to the presence of more or less incompressible fibrous areas and which represent an unequivocal sign of cellulite areas. In other words, the dedicated system of pressure 130 and revolution 132 sensors is adapted to ensure that, by placing the handpiece on the dermis only with its weight, the mechanical resistance encountered by the rotor balls while sliding on the patient's tissue is detected, thus detecting the type of normal, edematous or fibrous tissue with which the balls come into contact and therefore autonomously regulating the intensity of the device according to the resistance.

As shown in Fig. 2B, the electronic control unit 135 is located close to and behind the interface and in addition manages the power supply to said inductor device 140 through said connection device 131.

Another fundamental component of said inventive kit shown in Fig. 1 is represented by the presence in said base element 110 of an electrostimulator device connected via an electrical cable 260 to common electrodes 250, which may be self-adhesive electrodes, pre-gelled electrodes or an electrostimulating band, suitable for being positioned near the tissue portions of the body to be treated with the handpiece 150 and in which said electrical cable 260 is connected to the dedicated outlet 230 made on the base element 110 through a common inlet plug 261. The positioning of said electrodes 250 on the body areas of the patient represents a fundamental feature of the present invention, as it is inventively associated with the hypercompressive effect, determined by the presence of at least a row of rigid balls 198 between the rows with balls 190 having viscoelastic properties, allowing by virtue of a dedicated software regulating the parameters of the type of impulse, frequency, duration, intensity, to make the instant in which said hypercompression operated by the row of rigid balls 198 occurs on the tissue coincide with the instant in which the electrical impulse capable of producing the involuntary contraction of the muscle is generated. This synchronism is suitable for amplifying the active resistance of the muscle at the moment in which the hypercompressive effect occurs and therefore is capable of generating effective compressive and well-defined harmonic waves which propagate on the adjacent tissue.

A further and no less fundamental inventive feature relevant to the inventive purposes of the present kit concerns the possibility of carrying out a thermography through the presence of a digital thermographic camera 300 connected via a common electrical cable 310 (or via Wi-Fi or Bluetooth) to the dedicated outlet 330 made on said base element 110 by means of the inlet plug 361. The mapping of the portions of the body before, during and at the end of the treatment session, obtained by passing said digital thermographic camera 300 over the patient's body areas, allows to highlight both visually in real time and by storing the data in the form of a thermographic map, with different colors, the thermal differences that are generated on the tissue. In this way it is possible to differentiate normothermic areas, with normal blood circulation, from more hypothermic areas, where blood circulation is impaired or where there is poor circulation and therefore the presence of cellulite in its various stages. The data detected will be both displayed in real time by the operator, highlighting the areas where it is necessary to concentrate the activity of the device and sent to the computer terminal which will provide for drawing up a thermographic map which can be viewed on said screen 112. A further aspect, on the sidelines of all the technical solutions described up to now, but of particular relevance in the implementation of the invention, concerns the creation and development of a multiplicity of programs used in the data processing system and which create said software specially developed by the inventors, capable of being able to best and effectively perform all the functions deriving from the technical features described up to now. For this purpose said dedicated software is capable on the one hand of assisting the operator in real time by showing through said display 173 and said indicators 174 both the pressure exerted in real time by the rotor 200 and therefore exerted by the balls on the patient's tissue undergoing treatment and detected by said pressure sensor 130, and the number of revolutions of the rotor 200 detected in real time by the revolution sensor 132 and therefore to adjust the parameters of type of pulse, frequency, duration, so as to make the instant in which said hypercompression operated by the row of rigid balls 198 occurs on the tissue coincide with the instant in which the electric impulse is generated by means of the electrodes 250. Moreover, said dedicated software via the buttons 175 of the interface is adapted to allow the operator to be able to instantly vary all those parameters set at the origin of the session in the computer terminal on the basis of a standard basic setting or on the basis of the data present in the memory and obtained from the previous sessions of a specific patient. Specifically, the parameters set at the start of the session in the computer terminal are the speed of the rotor, the intensity of the impulses of the inductor, the impulse frequency and the feedback mechanism which links the automatic variation of the speed of the rotor on the basis of the values of resistance that the balls encounter during contact with the patient's tissue.

A further version of the present invention provides that the kit also includes an elastic band 340 adaptable to the body of any patient, comprising said electrodes 250. The function of the band 340 is to allow the treatment by the handpiece 150, avoiding damage or detachment of the electrodes 250 themselves.

It is therefore a specific object of the present invention to develop an effective medical kit including the relative dedicated software, capable of realizing the benefits deriving from a combined magnetotherapy treatment and a compressive microvibration treatment of the tissues and electrostimulation and all supported by the use of thermography in the treatment or alleviation of aesthetic disorders resulting from cellulite and all this through a non-invasive treatment and therefore suitable for all ages and not least without any contraindication.

A further and more general object is to be able to realize a combined magnetotherapy treatment with compressive microvibration and tissue electrostimulation capable of being advantageously used in the improvement of disorders associated with vascular hemodynamics, the increase in interstitial fibrous tissue, lymphatic stasis and lymphedema, to painful states resulting from edematous conditions, all obtainable with a few applications.

Of course, the data provided herein are purely illustrative and absolutely non-limiting of the scope of the present invention, since they serve exclusively to enable a person skilled in the art to understand some possible applications and embodiments of the invention. Therefore, from the foregoing it is clear that the inventive concept is adaptable to the particular needs of each individual case and that therefore different modifications can be made to the above description without departing from its scope of protection as defined by the following dependent claims.

## Claims

1. Kit for improving the disturbances associated with the inflammation of adipose tissue, with the phlebolymphatic pathologies and for muscular rehabilitation comprising a magnetotherapy device with compressive microvibration (100), an electrostimulator device and a digital thermographic camera (300) wherein said magnetotherapy device with compressive microvibration (100) comprises a base element (110) provided with at least a recess (111) suitable for housing at least a handpiece (150), with at least a screen (112) adapted to provide a communication interface between the user and the computer terminal comprised in said base element (110) and a system for electrical connection (120) that is wired or wireless, for the connection of said handpiece (150) to said base element (110), in that said electrostimulator device is housed inside said base element (110) and is connected by means of an electric cable (260) to common electrodes (250) adapted to be positioned in proximity to the body area affected by the treatment with said handpiece (150) and in which said electric cable (260) by means of a common inlet plug (261) is connected to a dedicated outlet (230) made on said base element (110), wherein said digital thermographic camera (300) is connected by means of a common electric cable (310) to a dedicated outlet (330) made on said base element (110) by means of an inlet plug (361), **characterized in that** said handpiece (150) is constituted by a head (160), by a body (170) and by a handle (180), in which in the head (160) and in the body (170) there is a recess adapted to house a rotor (200) adapted to carry out work in a radial direction with respect to an axis of an access hole made on said head (160), in which said rotor (200) has the form of a hollow cylinder, having on the lateral surface a plurality of balls (190) that are free to rotate on themselves and forming rows by means of the insertion of pins (192) in longitudinal central channels (191), in which each pin (192) forms a row to which at least four balls (190) belong and in which the axis of rotation of the balls (190) and of the rotor (200) correspond to that of insertion in the recess of the handpiece (150), in which said pins (192) are constrained respectively to a head ring nut (181) and to a ring nut of the handle (162), in which said ring nut of the handle (162) comprises a central fitting system adapted to be inserted on a rotation mechanism (184) constituting part of an electric motor (171) housed within said handle (180), in which said electric motor (171) is electrically connected, by means of isolating support means (420) and tightening means (410), to an electrical connection cable (120) connected to the computer terminal comprised in said base element (110), in which the rotation of the rotor (200) is such to cause the passive rotation of each single ball (190) on itself each time that this comes into contact with the tissue of the patient, by means of a rectangular opening (172) made on said body (170), in which on the body (170), in a position opposite that of the rectangular opening (172), an interface is attained comprising a display (173), a plurality of indicators (174) and a plurality of buttons (175), in which the head (160) comprises a flange (161) having one face adapted to allow the rotation of said head ring nut (181) and having the other face constrained to a cap (182), in which said cap (182) is connected to the head (160) by means of at least a common pair of reversible connection systems (183); wherein said balls (190) are configured with a different geometric solution as a function of the effect that one wishes to obtain; said balls (190) are made of elastomeric material with an internal structure constituted by a plurality of longitudinal internal channels (193) attained parallel around said longitudinal central channel (191), and the presence of said longitudinal internal channels (193) is adapted to confer to said balls (190) viscoelastic properties that are defined and exploited for controllably absorbing the pressure exerted by the handpiece (150) onto the tissue under treatment of the patient; wherein in the cavity of said rotor (200) there is an inducer device (140), constituted by at least a solenoid with cylindrical shape constrained at one end to a rotary bearing (141), and said rotary bearing (141) is constrained to said head ring nut (181); wherein on said cap (182) there is a pressure sensor (130) adapted to detect the pressure of the rotor (200) exerted by the balls (190) on the tissue of the patient during the treatment, and said pressure sensor (130) is connected with an electronic control unit (135) by means of a connection device (131), constituted by a connector provided with a plurality of pins; wherein in proximity to the rotation mechanism (184) there is a revolution sensor (132) or an encoder positioned on the rotary axis of the motor, adapted to detect the revolutions of the rotor (200) and to send the data to said electronic control unit (135); wherein said electronic control unit (135) is controlled by said computer terminal by means of a wiring system that passes by means of said electrical connection system (120) with or without wires, and said electronic control unit (135) is situated in proximity to and behind the interface and manages, by means of said connection device (131), the power supply to said inducer device (140).

2. Kit according to claim 1, **characterized in that** said balls (190) belonging to each row have the same viscoelastic property, and **in that** in said rotor (200) there is at least a row of rigid balls (198) lacking said longitudinal internal channels (193) and adapted to carry out a hyper-compressive effect suitable for exploiting the muscle as active resistance, at the time when they cyclically come into contact with the body tissue and **in that** the presence of the rigid balls (198) and of the elastomeric balls (190) during the rotation of the rotor is adapted to produce compressive harmonic waves.

3. Kit according to any one of the preceding claims, **characterized in that** the presence of the pressure sensor (130) and of the revolution sensor (132) is adapted to allow the detection of the mechanical resistance encountered by the rigid balls (198) and by the balls (190) during the sliding on the tissue of the patient and **in that** said resistance is adapted to indicate which type of tissue, normal, edematous or fibrous, is in contact with the aforesaid balls and **in that** by means of said display (173) and said indicators (174), the pressure is indicated which is exerted by the rotor (200) on the tissue of the patient subjected to therapy and detected by said pressure sensor (130).

4. Kit according to any one of the preceding claims comprising a multiplicity of programs employed in the data processing system and attaining a dedicated software, in which said dedicated software is **characterized in that** it is adapted to self-regulate in real time the speed of the rotor (200) and the intensity of the pulses of the inducer device (140) based on the values of the resistance that the balls encounter during the contact with the tissue of the patient and detected by said pressure sensor (130) and by the revolution sensor (132).

5. Kit according to any one of the preceding claims **characterized in that** said dedicated software is adapted to self-regulate in real time the speed of the rotor (200) and the frequency, duration, intensity of the electric pulse actuated by the electrostimulator by means of said common electrodes (250) positioned in proximity to the body area subjected to the action of the handpiece (150), **in that** said dedicated software makes the instant at which there occurs said hypercompression, operated by the row of rigid balls (198) on the tissue, coincide with the instant at which the electric pulse is generated which causes an involuntary contraction of the muscle and **in that** said synchronism is adapted to amplify the active resistance of the muscle at the time in which there is the hypercompressive effect, generating compressive harmonic waves propagating on the neighboring tissue.

6. Kit according to any one of the preceding claims **characterized in that** said electrostimulator is connected to the electrodes (250) and **in that** belonging to said electrodes (250) are the self-adhesive electrodes, the pre-gelled electrodes and those constituting part of an electrostimulator band.

7. Kit according to any one of the preceding claims **characterized in that** said dedicated software is adapted to be configured by the operator at the start of the session by means of the computer terminal and **in that** said configuration occurs based on the data present in the memory and obtained from the preceding sessions of the patient and **in that** said dedicated software is adapted to be configured by the operator at the start of the session so as to set the value of the speed of the rotor (200) and the intensity of the pulses of the inducer device (140).

8. Kit according to any one of the preceding claims **characterized in that** said dedicated software is adapted to allow the operator, by means of the buttons (175) of the interface, to vary in real time the parameters set at the start of the session in the computer terminal.

9. Kit according to any one of the preceding claims **characterized in that** the thermographic data detected by said digital thermographic camera (300) on the body areas of the patient, before, during and at the end of the treatment session, is adapted to be stored in said computer terminal and **in that** by means of said data the dedicated software is adapted to process a thermographic map showing the hypovascularization zones.

10. Kit according to any one of the preceding claims **characterized in that** it comprises an elastic band (340) adaptable to the body of any one patient, said band (340) comprising said electrodes (250) and being adapted to allow the treatment by said handpiece (150), avoiding the damage or the detachment of the electrodes (250) themselves.

## Patentansprüche

1. Kit zur Verbesserung der Störungen im Zusammenhang mit Entzündungen des Fettgewebes, mit phlebolymphatischen Erkrankungen und zur Muskelrehabilitation, umfassend eine Magnettherapieeinrichtung mit komprimierender Mikrovibration (100), eine Elektrostimulationseinrichtung und eine digitale Thermografiekamera (300), wobei die Magnettherapieeinrichtung mit komprimierender Mikrovibration (100) ein Basiselement (110) aufweist, das mit zumindest einer Aussparung (111) versehen ist, die zur Aufnahme zumindest eines Handstücks (150) geeignet ist, mit zumindest einem Bildschirm (112), der dazu angepasst ist, eine Kommunikationsschnittstelle zwischen dem Benutzer und dem in dem Basiselement (110) enthaltenen Computerterminal bereitzustellen, und einem System zum elektrischen Anschluss (120), das drahtgebunden oder drahtlos ist, zum Anschluss des Handstücks (150) an das Basiselement (110), wobei die Elektrostimulationseinrichtung in dem Basiselement (110) untergebracht und mittels eines elektrischen Kabels (260) mit üblichen Elektroden (250) verbunden ist, die dazu angepasst sind, in der Nähe des von der Behandlung mit dem Handstück (150) betroffenen Körperbereichs positioniert zu werden und in denen das elektrische Kabel (260) mittels eines üblichen Eingangssteckers (261) mit einem zugehörigen Ausgang (230), der an dem Basiselement (110) ausgebildet ist, verbunden ist, wobei die digitale Thermografiekamera (300) mittels eines üblichen elektrischen Kabels (310) mittels eines Eingangssteckers (361) mit einem zugehörigen Ausgang (330), der an dem Basiselement (110) ausgebildet ist, verbunden ist, **dadurch gekennzeichnet, dass** das Handstück (150) durch einen Kopf (160), einen Körper (170) und einen Griff (180) gebildet ist, wobei in dem Kopf (160) und in dem Körper (170) eine Aussparung vorhanden ist, die dazu angepasst ist, einen Rotor (200) aufzunehmen, der dazu angepasst ist, eine Arbeit in einer radialen Richtung in Bezug auf eine Achse eines in dem Kopf (160) ausgebildeten Zugangslochs auszuführen, wobei der Rotor (200) die Form eines Hohlzylinders aufweist, der an der Seitenfläche mehrere Kugeln (190) aufweist, die frei um sich selbst drehbar sind und durch das Einführen von Stiften (192) in längliche zentrale Kanäle (191) Reihen bilden, wobei jeder Stift (192) eine Reihe bildet, zu der zumindest vier Kugeln (190) gehören, und wobei die Drehachse der Kugeln (190) und des Rotors (200) der Einführungsachse in die Aussparung des Handstücks (150) entsprechen, wobei die Stifte (192) jeweils an eine Kopfringmutter (181) und an eine Ringmutter des Griffs (162) gebunden sind, wobei die Ringmutter des Griffs (162) ein zentrales Passungssystem aufweist, das dazu angepasst ist, auf einen Drehmechanismus (184) eingesetzt zu werden, der einen Teil eines in dem Griff (180) untergebrachten Elektromotors (171) bildet, wobei der Elektromotor (171) mittels isolierender Stützmittel (420) und Befestigungsmitteln (410) elektrisch mit einem elektrischen Verbindungskabel (120) verbunden ist, das mit dem in dem Basiselement (110) enthaltenen Computerterminal verbunden ist, wobei die Drehung des Rotors (200) dergestalt ist, dass sie die passive Drehung jeder einzelnen Kugel (190) um sich selbst bewirkt, und zwar jedes Mal, wenn diese mit dem Gewebe des Patienten in Kontakt kommt, und zwar mittels einer rechteckigen Öffnung (172), die an dem Körper (170) gefertigt ist, wobei an dem Körper (170) in einer Position gegenüber der rechteckigen Öffnung (172) eine Schnittstelle erreicht wird, die eine Anzeige (173), mehrere Anzeiger (174) und mehrere Tasten (175) aufweist, wobei der Kopf (160) einen Flansch (161) aufweist, der eine Seite aufweist, die dazu angepasst ist, die Drehung der Kopfringmutter (181) zu ermöglichen, und die andere Seite aufweist, die an einer Kappe (182) gebunden ist, wobei die Kappe (182) mittels zumindest eines üblichen Paares reversibler Verbindungssysteme (183) mit dem Kopf (160) verbunden ist; wobei die Kugeln (190) als Funktion der gewünschten Wirkung, die jemand erzielen möchte, mit einer unterschiedlichen geometrischen Lösung gebildet sind; wobei die Kugeln (190) aus elastomerem Material mit einer inneren Struktur bestehen, die durch mehrere längliche innere Kanäle (193) gebildet ist, die parallel um den länglichen zentralen Kanal (191) herum ausgebildet sind, und das Vorhandensein der länglichen inneren Kanäle (193) dazu dient, den Kugeln (190) viskoelastische Eigenschaften zu verleihen, die definiert sind und genutzt werden, um den durch das Handstück (150) auf das behandelte Gewebe des Patienten ausgeübten Druck kontrollierbar zu absorbieren; wobei in dem Hohlraum des Rotors (200) eine Erregereinrichtung (140) vorhanden ist, die durch zumindest ein Solenoid mit zylindrischer Form gebildet ist, das an einem Ende an ein Drehlager (141) gebunden ist, und das Drehlager (141) an die Kopfringmutter (181) gebunden ist; wobei auf der Kappe (182) ein Drucksensor (130) vorhanden ist, der dazu angepasst ist, den Druck des Rotors (200) zu ermitteln, der durch die Kugeln (190) während der Behandlung auf das Gewebe des Patienten ausgeübt wird, und wobei der Drucksensor (130) mittels einer Verbindungseinrichtung (131), die durch einen mit mehreren Stiften versehenen Verbinder gebildet ist, mit einer elektronischen Steuereinheit (135) verbunden ist; wobei in der Nähe des Drehmechanismus (184) auf der Drehachse des Motors ein Umdrehungssensor (132) oder ein Encoder angeordnet ist, der dazu angepasst ist, die Umdrehungen des Rotors (200) zu ermitteln und die Daten an die elektronische Steuereinheit (135) zu senden; wobei die elektronische Steuereinheit (135) durch das Computerterminal mittels eines Verkabelungssystems gesteuert wird, das mittels des elektrischen Verbindungssystems (120) drahtgebunden oder drahtlos verläuft, und die elektronische Steuereinheit (135) in der Nähe und hinter der Schnittstelle angeordnet ist und mittels der Verbindungseinrichtung (131) die Leistungszufuhr an die Erregereinrichtung (140) steuert.

2. Kit gemäß Anspruch 1, **dadurch gekennzeichnet, dass** die zu jeder Reihe gehörenden Kugeln (190) die gleiche viskoelastische Eigenschaft aufweisen und dass in dem Rotor (200) zumindest eine Reihe starrer Kugeln (198) vorhanden ist, die keine länglichen inneren Kanäle (193) aufweisen und dazu angepasst sind, eine hyperkomprimierende Wirkung auszuüben, die geeignet ist, den Muskel als aktiven Widerstand zu nutzen, wenn sie zyklisch mit dem Körpergewebe in Kontakt kommen, und dass das Vorhandensein der starren Kugeln (198) und der elastomeren Kugeln (190) während der Drehung des Rotors dazu angepasst ist, komprimierende harmonische Wellen zu erzeugen.

3. Kit gemäß einem der vorangehenden Ansprüche, **dadurch gekennzeichnet, dass** das Vorhandensein des Drucksensors (130) und des Umdrehungssensors (132) dazu angepasst ist, die Ermittlung des mechanischen Widerstands zu ermöglichen, auf den die starren Kugeln (198) und die Kugeln (190) beim Gleiten auf dem Gewebe des Patienten treffen, und dadurch, dass der Widerstand dazu angepasst ist, anzuzeigen, welche Art von Gewebe, nämlich normales, ödematöses oder fibröses Gewebe, mit den vorgenannten Kugeln in Kontakt steht, und dadurch, dass mittels der Anzeige (173) und der Anzeiger (174) der Druck angezeigt wird, der durch den Rotor (200) auf das Gewebe des Patienten, der der Therapie unterzogen wird, ausgeübt und durch den Drucksensor (130) ermittelt wird.

4. Kit nach einem der vorangehenden Ansprüche, der eine Mehrzahl von Programmen aufweist, die in dem Datenverarbeitungssystem verwendet werden und eine spezielle Software erzielen, wobei die spezielle Software **dadurch gekennzeichnet ist, dass** sie dazu angepasst ist, die Geschwindigkeit des Rotors (200) und die Intensität der Impulse der Erregereinrichtung (140) basierend auf den Werten des Widerstands, den die Kugeln während des Kontakts mit dem Gewebe des Patienten erfahren und der durch den Drucksensor (130) und den Umdrehungssensor (132) erfasst wird, in Echtzeit selbst zu regulieren.

5. Kit gemäß einem der vorangehenden Ansprüche, **dadurch gekennzeichnet, dass** die spezielle Software dazu angepasst ist, die Geschwindigkeit des Rotors (200) und die Frequenz, Dauer und Intensität des elektrischen Pulses, der durch den Elektrostimulator mittels der üblichen Elektroden (250), die in der Nähe des Körperbereichs, der der Aktion des Handstücks (150) unterzogen wird, angeordnet sind, veranlasst wird, in Echtzeit selbst zu regulieren, und dadurch, dass die spezielle Software den Zeitpunkt, zu dem die Hyperkomprimierung auftritt, betätigt durch die Reihe starrer Kugeln (198) auf dem Gewebe, mit dem Zeitpunkt zusammenfallen lässt, zu dem der elektrische Puls erzeugt wird, der eine unwillkürliche Kontraktion des Muskels bewirkt, und dadurch, dass dieser Synchronismus dazu angepasst ist, den aktiven Widerstand des Muskels zu der Zeit, zu der der hyperkomprimierende Effekt vorliegt, zu verstärken, wodurch auf das benachbarte Gewebe ausbreitende komprimierende harmonische Wellen erzeugt werden.

6. Kit gemäß einem der vorangehenden Ansprüche, **dadurch gekennzeichnet, dass** der Elektrostimulator mit den Elektroden (250) verbunden ist und dass zu den Elektroden (250) die selbstklebenden Elektroden, die vorgegelten Elektroden und solche, die Teil eines Elektrostimulatorbandes sind, gehören.

7. Kit gemäß einem der vorangehenden Ansprüche, **dadurch gekennzeichnet, dass** die spezielle Software dazu angepasst ist, dass durch den Bediener zu Beginn der Sitzung mittels des Computerterminals konfiguriert zu werden, und dass die Konfiguration basierend auf den Daten erfolgt, die in dem Speicher vorhandenen sind und aus den vorangegangenen Sitzungen des Patienten gewonnen wurden, und dadurch, dass die spezielle Software dazu angepasst ist, zu Beginn der Sitzung durch den Bediener konfiguriert zu werden, um den Wert der Geschwindigkeit des Rotors (200) und die Intensität der Pulse der Erregereinrichtung (140) einzustellen.

8. Kit gemäß einem der vorangehenden Ansprüche, **dadurch gekennzeichnet, dass** die spezielle Software dazu angepasst ist, es dem Bediener zu ermöglichen, mittels der Tasten (175) der Schnittstelle die zu Beginn der Sitzung in dem Computerterminal eingestellten Parameter in Echtzeit zu ändern.

9. Kit gemäß einem der vorangehenden Ansprüche, **dadurch gekennzeichnet, dass** die durch die digitale Thermografiekamera (300) vor, während und am Ende der Behandlungssitzung an den Körperbereichen des Patienten ermittelten thermografischen Daten dazu angepasst sind, in dem Computerterminal gespeichert zu werden, und dadurch, dass die spezielle Software mittels der Daten dazu angepasst ist, eine thermografische Karte zu verarbeiten, die die Hypovaskularisationszonen anzeigt.

10. Kit gemäß einem der vorangehenden Ansprüche, **dadurch gekennzeichnet, dass** es ein elastisches Band (340) aufweist, das an den Körper eines beliebigen Patienten angepasst werden kann, wobei das Band (340) die Elektroden (250) aufweist und dazu angepasst ist, die Behandlung durch das Handstück (150) zu ermöglichen und dabei die Beschädigung oder das Ablösen der Elektroden (250) selbst zu vermeiden.

## Revendications

1. Kit pour améliorer les troubles associés à l'inflammation du tissu adipeux, aux pathologies phlébolymphatiques et pour la rééducation musculaire comprenant un dispositif de magnétothérapie à microvibration compressive (100), un dispositif d'électrostimulation et une caméra thermographique numérique (300) dans lequel ledit dispositif de magnétothérapie à microvibration compressive (100) comprend un élément de base (110) pourvu d'au moins un évidement (111) apte à loger au moins une pièce à main (150), avec au moins un écran (112) adapté pour fournir une interface de communication entre l'utilisateur et le terminal informatique compris dans ledit élément de base (110) et un système pour une connexion électrique (120) qui est câblé ou sans fil, pour la connexion de ladite pièce à main (150) audit élément de base (110), en ce que ledit dispositif d'électrostimulation est logé à l'intérieur dudit élément de base (110) et est connecté à l'aide d'un câble électrique (260) à des électrodes communes (250) adaptées pour être positionnées à proximité de la zone corporelle affectée par le traitement avec ladite pièce à main (150) et dans lequel ledit câble électrique (260) est connecté à l'aide d'une fiche d'entrée commune (261) à une sortie dédiée (230) réalisée sur ledit élément de base (110), dans lequel ladite caméra thermographique numérique (300) est connectée à l'aide d'un câble électrique commun (310) à une sortie dédiée (330) réalisée sur ledit élément de base (110) à l'aide d'une fiche d'entrée (361),
**caractérisé en ce que** ladite pièce à main (150) est constituée d'une tête (160), d'un corps (170) et d'une poignée (180), dans lequel il y a dans la tête (160) et dans le corps (170) un évidement adapté pour loger un rotor (200) adapté pour effectuer un travail dans une direction radiale par rapport à un axe d'un trou d'accès réalisé sur ladite tête (160), dans lequel ledit rotor (200) présente la forme d'un cylindre creux, présentant sur la surface latérale une pluralité de billes (190) qui sont libres de tourner sur elles-mêmes et formant des rangées à l'aide de l'insertion de goupilles (192) dans des canaux centraux longitudinaux (191), dans lequel chaque goupille (192) forme une rangée dont font partie au moins quatre billes (190) et dans lequel l'axe de rotation des billes (190) et du rotor (200) correspond à celui d'insertion dans l'évidement de la pièce à main (150), dans lequel lesdites goupilles (192) sont contraintes respectivement sur un écrou à œil de tête (181) et sur un écrou à œil de la poignée (162), dans lequel ledit écrou à œil de la poignée (162) comprend un système de raccord central adapté pour être inséré sur un mécanisme de rotation (184) constituant une partie d'un moteur électrique (171) logé à l'intérieur de ladite poignée (180), dans lequel ledit moteur électrique (171) est connecté électriquement, à l'aide de moyens de support isolants (420) et de moyens de serrage (410), à un câble de connexion électrique (120) connecté au terminal informatique compris dans ledit élément de base (110), dans lequel la rotation du rotor (200) est de nature à provoquer la rotation passive de chaque bille (190) sur elle-même à chaque fois que celle-ci entre en contact avec le tissu du patient, à l'aide d'une ouverture rectangulaire (172) réalisée sur ledit corps (170), dans lequel sur le corps (170), dans une position opposée à celle de l'ouverture rectangulaire (172), une interface est atteinte comprenant un affichage (173), une pluralité d'indicateurs (174) et une pluralité de boutons (175), dans lequel la tête (160) comprend une bride (161) présentant une face adaptée pour permettre la rotation dudit écrou à œil de tête (181) et présentant l'autre face contrainte sur un capuchon (182), dans lequel ledit capuchon (182) est relié à la tête (160) à l'aide d'au moins une paire commune de systèmes de connexion réversibles (183) ;
dans lequel lesdites billes (190) sont configurées avec une solution géométrique différente en fonction de l'effet que l'on souhaite obtenir ; lesdites billes (190) sont en matériau élastomère avec une structure interne constituée par une pluralité de canaux internes longitudinaux (193) atteints parallèlement autour dudit canal central longitudinal (191), et la présence desdits canaux internes longitudinaux (193) est adaptée pour conférer auxdites billes (190) des propriétés viscoélastiques qui sont définies et exploitées pour absorber de manière contrôlable la pression exercée par la pièce à main (150) sur le tissu sous traitement du patient ; dans lequel il y a dans la cavité dudit rotor (200) un dispositif inducteur (140), constitué par au moins un solénoïde de forme cylindrique contraint à une extrémité sur un palier rotatif (141), et ledit palier rotatif (141) est contraint sur ledit écrou à œil de tête (181) ; dans lequel il y a sur ledit capuchon (182) un capteur de pression (130) adapté pour détecter la pression du rotor (200) exercée par les billes (190) sur le tissu du patient pendant le traitement, et ledit capteur de pression (130) est connecté à une unité de commande électronique (135) à l'aide d'un dispositif de connexion (131) constitué d'un connecteur doté d'une pluralité de goupilles ; dans lequel à proximité du mécanisme de rotation (184) il y a un capteur de révolution (132) ou un encodeur positionné sur l'axe de rotation du moteur, adapté pour détecter les révolutions du rotor (200) et pour envoyer les données à ladite unité de commande électronique (135) ; dans lequel ladite unité de commande électronique (135) est commandée par ledit terminal informatique à l'aide d'un système de câblage qui passe à l'aide dudit système de connexion électrique (120) avec ou sans fils, et ladite unité de commande électronique (135) est située à proximité de l'interface et derrière celle-ci et gère, à l'aide dudit dispositif de connexion (131), l'alimentation électrique dudit dispositif inducteur (140).

2. Kit selon la revendication 1, **caractérisé en ce que** lesdites billes (190) faisant partie de chaque rangée présentent la même propriété viscoélastique, et **en ce qu'**il y a dans ledit rotor (200) au moins une rangée de billes rigides (198) dépourvues desdits canaux internes longitudinaux (193) et adaptées pour effectuer un effet hyper-compressif apte à exploiter le muscle comme résistance active, au moment où elles entrent cycliquement en contact avec le tissu corporel et **en ce que** la présence des billes rigides (198) et des billes élastomères (190) pendant la rotation du rotor est adaptée pour produire des ondes harmoniques de compression.

3. Kit selon l'une des revendications précédentes, **caractérisé en ce que** la présence du capteur de pression (130) et du capteur de révolution (132) est adaptée pour permettre la détection de la résistance mécanique rencontrée par les billes rigides (198) et par les billes (190) lors du glissement sur le tissu du patient et **en ce que** ladite résistance est adaptée pour indiquer quel type de tissu, normal, œdémateux ou fibreux, est en contact avec lesdites billes et **en ce qu'**à l'aide dudit affichage (173) et desdits indicateurs (174), la pression qui est exercée par le rotor (200) sur le tissu du patient soumis au traitement et détectée par ledit capteur de pression (130) est indiquée.

4. Kit selon l'une des revendications précédentes comprenant une multiplicité de programmes employés dans le système de traitement de données et atteignant un logiciel dédié, dans lequel ledit logiciel dédié est **caractérisé en ce qu'**il est adapté pour autoréguler en temps réel la vitesse du rotor (200) et l'intensité des impulsions du dispositif inducteur (140) sur la base des valeurs de la résistance que rencontrent les billes lors du contact avec le tissu du patient et détectées par ledit capteur de pression (130) et par le capteur de révolution (132).

5. Kit selon l'une des revendications précédentes **caractérisé en ce que** ledit logiciel dédié est adapté pour autoréguler en temps réel la vitesse du rotor (200) et la fréquence, la durée, l'intensité de l'impulsion électrique actionnée par l'électrostimulateur à l'aide desdites électrodes communes (250) positionnées à proximité de la zone corporelle soumise à l'action de la pièce à main (150), **en ce que** ledit logiciel dédié fait coïncider l'instant où se produit ladite hypercompression, mise en œuvre par la rangée de billes rigides (198) sur le tissu, avec l'instant où l'impulsion électrique est générée ce qui provoque une contraction involontaire du muscle et **en ce que** ledit synchronisme est adapté pour amplifier la résistance active du muscle au moment où il y a l'effet hypercompressif, générant des ondes harmoniques de compression se propageant sur le tissu voisin.

6. Kit selon l'une des revendications précédentes **caractérisé en ce que** ledit électrostimulateur est connecté aux électrodes (250) et **en ce que** les électrodes auto-adhésives, les électrodes prégélifiées et celles constituant une partie d'une bande d'électrostimulation font partie desdites électrodes (250).

7. Kit selon l'une des revendications précédentes **caractérisé en ce que** ledit logiciel dédié est adapté pour être configuré par l'opérateur au début de la session à l'aide du terminal informatique et **en ce que** ladite configuration intervient sur la base des données présentes dans la mémoire et obtenues à partir des sessions précédentes du patient et **en ce que** ledit logiciel dédié est adapté pour être configuré par l'opérateur au début de la session de manière à régler la valeur de la vitesse du rotor (200) et l'intensité des impulsions du dispositif inducteur (140).

8. Kit selon l'une des revendications précédentes **caractérisé en ce que** ledit logiciel dédié est adapté pour permettre à l'opérateur, à l'aide des boutons (175) de l'interface, de faire varier en temps réel les paramètres réglés au début de la session dans le terminal informatique.

9. Kit selon l'une des revendications précédentes **caractérisé en ce que** les données thermographiques détectées par ladite caméra thermographique numérique (300) sur les zones corporelles du patient, avant, pendant et à la fin de la session de traitement, sont adaptées pour être stockées dans ledit terminal informatique et **en ce qu'**à l'aide desdites données le logiciel dédié est adapté pour traiter une carte thermographique montrant les zones d'hypovascularisation.

10. Kit selon l'une des revendications précédentes **caractérisé en ce qu'**il comprend une bande élastique (340) adaptable au corps d'un patient quelconque, ladite bande (340) comprenant =- lesdites électrodes (250) et étant adaptée pour permettre le traitement par ladite pièce à main (150), évitant l'endommagement ou le détachement des électrodes (250) elles-mêmes.
